# EUROPEAN PATENT SPECIFICATION

(11) **EP 4 231 996 B1**
(45) Date of publication and mention of the grant of the patent: **18.09.2024**
(21) Application number: 21789856.8
(22) Date of filing: 29.09.2021
(51) Int. Cl.: A61K 9/08, A61K 31/137

(54) **READY TO USE, NORADRENALINE DRIP BAGS, HAVING LOW SUBVISIBLE PARTICLE COUNTS**
GEBRAUCHSFERTIGE NORADRENALIN-TROPFBEUTEL MIT GERINGEN ANZAHLEN SUBSICHTBARER PARTIKEL
POCHES DE PERFUSION PRÊTES À L'EMPLOI, À FAIBLE NOMBRE DE PARTICULES SUBVISIBLES

(30) Priority: 21.10.2020 US 202063094378 P
(43) Date of publication of application: 30.08.2023
(73) Proprietor: Sintetica S.A., 6850 Mendrisio (CH)
(72) Inventor: CARONZOLO, Nicola, 6850 Mendrisio (CH); DONATI, Elisabetta, 6850 Mendrisio (CH); BIANCHI, Clara, 6850 Mendrisio (CH)
(74) Representative: Hoefer & Partner Patentanwälte mbB
(86) International application number: PCT/IB2021/058972
(87) International publication number: WO 2022/084775

(56) References cited:
- US-A1- 2005 070 613
- US-A1- 2016 058 715
- US-A1- 2017 049 720
- US-A1- 2018 043 020
- US-B2- 10 251 848
- US-B2- 10 653 646

## Description

### FIELD OF THE INVENTION

The present invention as defined in the appended claims relates to ready to use noradrenaline drug products having low subvisible particle concentrations, and to materials and methods useful for the prevention of subvisible particle formation.

### BACKGROUND OF THE INVENTION

Noradrenaline (N-desmethylepinephrine) (a/k/a norepinephrine) is a potent α-sympathomimetic drug and physiological neurotransmitter with the typical core structure of the phenylalkylamines (2-phenyl-1-aminoethane). The hydrogen tartrate and hydrochloride salts are widely used in injectable solutions for acute treatment of hypotension, anaphylactic shocks, and as vasoconstrictive additives in local anesthetic formulations to prolong the analgesic effect. Due to their common use in emergency medicine and intensive care units (ICUs), the typical dosage forms are ampoules for single use and sterile injectable solutions in glass bottles.

Manufacturers and pharmacy compounders take several precautions to control noradrenaline degradation during the drug product's shelf life including refrigeration and protection from light. Refrigeration, however, is an inconvenient process particularly considering the opportunities for transgressing temperature limits during distribution and use of the product. Protection from light is more practical from the manufacturer's perspective, but it inhibits inspection of the product, such that visible degradation cannot be discovered until the product is actually opened and put to use. Examples of light protection are disclosed by Yadav (WO 2018/140894 A1) and Rakesh (US 2016/0058715 A1), which employed aluminum foil over-wraps to protect their dilute noradrenaline solutions from light.

Manufacturers also add antioxidants and chelating agents to noradrenaline formulations to help stabilize them. Hospira Inc., for example, markets a 5 ml ampoule comprising 1 mg/ml noradrenaline bitartrate in the United States that contains sodium metabisulfite under the brand name Levophed^{®}. The label specifically cautions that sodium metabisulfite "may cause allergic-type reactions including anaphylactic symptoms and life-threatening or less severe asthmatic episodes in certain susceptible people." Yadav (WO 2018/140894 A1) and Rakesh (US 2016/0058715 A1) disclose high volume ready to use bags of noradrenaline bitartrate and propose EDTA as an oxygen scavenger.

Still others such as Dinnequin (U.S. Pub. No. 2005/0070613 A1) try to control degradation by strictly controlling oxygen during the manufacturing process. Nevertheless, in spite of several strict safeguards against oxygen incursion, Dinnequin reported that "Tests carried out at 25° C under light showed the presence of a coloured precipitate from the 5th day and an impurities concentration higher than 3% including especially adenochrome."

Mitidieri (U.S. Patent No. 10,251,848) discusses the relevance of pH to the stability of noradrenaline, in addition to the need to limit oxygen concentrations in the finished product. These vials are tightly controlled for oxygen in the headspace and solution, and have excellent stability against product degradation. In Europe, the patent's owner (Sintetica S.A.) has sold 50 ml clear glass vials having noradrenaline concentrations in the range of 0.04-0.2 mg/ml, corresponding to the ones disclosed in Patent No. 10,251,848.

While mechanisms of noradrenaline degradation have received much attention over the years, (see, e.g. Hoellein et al., International Journal of Pharmaceutics 434 (2012) 468-480), very little attention has been given to subvisible particles, and mechanisms for their control. This is in spite of the United States Pharmacopoeia, which imposes limits on subvisible particles in injectable solutions in chapter <788>.

Traun et al., International Journal of Pharmacy Compounding, Vol. 10 No. 3, May/June 2006, reports that subvisible particles in sterile injectable products can originate from many sources, including the solution itself and its ingredients, the production process and its variables (e.g., environment, equipment, personnel), the product's packaging, and the preparation of the product for administration (e.g., manipulating the product, the environment in which it is prepared). However, very little has been published on the formation of subvisible particles by solutions of noradrenaline.

What is needed are new methods and formulations for controlling the stability of noradrenaline injectable drug products, particularly for controlling the generation of subvisible particles in dilute ready to use noradrenaline liquid solutions.

### SUMMARY OF THE INVENTION

During their investigation into the conditions responsible for subvisible particles in noradrenaline solutions, the inventors have made several important discoveries that enable, for the first time, dilute ready to use (RTU) bags of noradrenaline without any requirement for light protection, chelating agents, or antioxidants. While subvisible particles in dilute noradrenaline solutions do not appear to result from oxidation, as their formation occurs at concentrations below which oxidative degradation is observed, the concentration of oxygen in the headspace does affect their formation. Consequently, the production of subvisible particles can be controlled by limiting the amount of oxygen in the headspace of the drug product to levels below which no oxidative degradation can be observed.

The inventors have also discovered a protective influence from translucent and transparent plastic materials that prevents the formation of subvisible particles, even when the plastic is not an excellent barrier to oxygen, and even when oxygen concentrations in the solution exceed those found in glass containers. Without wishing to be bound by any theory, it is believed that the light transmission properties of plastics such as translucent polypropylene helps to protect the solution from light rays responsible the formation of subvisible particles, while keeping the container clear for human observation. The invention has particular applicability to plastics having optical properties comparable to a translucent polypropylene or a translucent film comprising polypropylene, ethylene vinyl alcohol (27 mol % ethylene), and polypropylene plies.

Thus, in a first principal embodiment the invention provides a hermetically sealed noradrenaline drug product in an intravenous ready to use translucent or transparent collapsible drip bag having low subvisible particle counts comprising: (a) a headspace consisting essentially of an inert gas and < 2% v/v oxygen; and (b) a liquid solution comprising: (i) noradrenaline or a pharmaceutically acceptable salt thereof at a concentration of from 0.001 to 0.2 mg/ml or from 0.001 to 0.035 mg/ml, (ii) less than 600 ppbw oxygen, (iii) ≤ 25 per mL subvisible particles equal to or greater than 10 µm, and (iv) ≤ 3 per mL subvisible particles equal to or greater than 25 µm; wherein: (i) the concentration of noradrenaline or pharmaceutically acceptable salt is based on the weight of the free base of noradrenaline (ii) the subvisible particle count is determined by the light obscuration particle count test in USP <788> (May 1, 2013); (iii) the oxygen content in the headspace and solution is measured immediately after the product is hermetically sealed or after the oxygen content in the headspace and solution has reached equilibrium (iv) the particle counts are observed immediately after the product is hermetically sealed or after the oxygen content in the headspace and solution has reached equilibrium; (v) the drug product is free of antioxidants and chelating agents.

In a second more particular embodiment, the invention provides a hermetically sealed noradrenaline drug product in an intravenous ready to use translucent or transparent collapsible drip bag having low subvisible particle counts comprising: (a) a headspace consisting essentially of an inert gas, < 1.5% v/v oxygen immediately after the bag is hermetically sealed, and < 1.2% v/v after the oxygen in the headspace and solution have reached equilibrium; and (b) an aqueous liquid solution comprising: (i) noradrenaline or a pharmaceutically acceptable salt thereof at a concentration of from 0.001 to 0.2 mg/ml or from 0.001 to 0.035 mg/ml, (ii) a tonicifying effective amount of sodium chloride, (iii) hydrochloric acid in an amount effective to impart a pH of from 3.0 to 4.5, (iv) less than 200 ppbw oxygen immediately after the product has been hermetically sealed, (v) less than 500 ppbw oxygen after the oxygen in the solution and headspace have reached equilibrium, (vi) ≤ 25 per mL subvisible particles equal to or greater than 10 µm, and (vii) ≤ 3 per mL subvisible particles equal to or greater than 25 µm; wherein: (i) the concentration of noradrenaline or pharmaceutically acceptable salt is based on the weight of the free base of noradrenaline; (ii) the subvisible particle count is determined by the light obscuration particle count test in USP <788> (May 1, 2013); (iii) the particle counts are observed immediately after the product is hermetically sealed and after the oxygen content in the headspace and solution has reached equilibrium; and (iv) the drug product is free of chelating agents and antioxidants.

Another principal embodiment relates to methods of making the bags of the current invention, and bags made thereby. Thus, in a third principal embodiment the invention provides a method of making a hermetically sealed noradrenaline drug product in an intravenous ready to use translucent or transparent collapsible drip bag having low subvisible particle counts comprising: (a) dissolving noradrenaline or a pharmaceutically acceptable salt thereof in water to produce a noradrenaline liquid solution at a concentration of from 0.001 to 0.2 or from 0.001 to 0.035 mg/ml; (b) distributing the noradrenaline solution and an inert gas into the bag, such that the percentage of the bag's volume occupied by the headspace is from 0 to 25% or from 2 to 15%; and (c) hermetically sealing the bag; wherein: (i) the oxygen concentration in the solution is less than 200 ppbw immediately after sealing the container and less than 500 ppbw after the oxygen content in the headspace and solution have reached equilibrium; (ii) the oxygen concentration in the headspace is less than 1.5 % immediately after sealing the container and less than 1.2% after the oxygen content in the headspace and solution have reached equilibrium; (iii) the drug product omits chelating agents, preservatives, and antioxidants; and (iv) when at equilibrium with the headspace, the oxygen content in the solution is less than 500 ppbw.

Additional advantages of the invention are set forth in part in the description which follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### BRIEF DESCRIPTION OF THE FIGURES

The patent or application file contains at least one drawing executed in color. Copies of this patent or patent application publication with color drawing(s) will be provided by the Office upon request and payment of the necessary fee.

The accompanying drawings, which are incorporated in and constitute a part of this specification, illustrate several embodiments of the invention and together with the description serve to explain the principles of the invention.

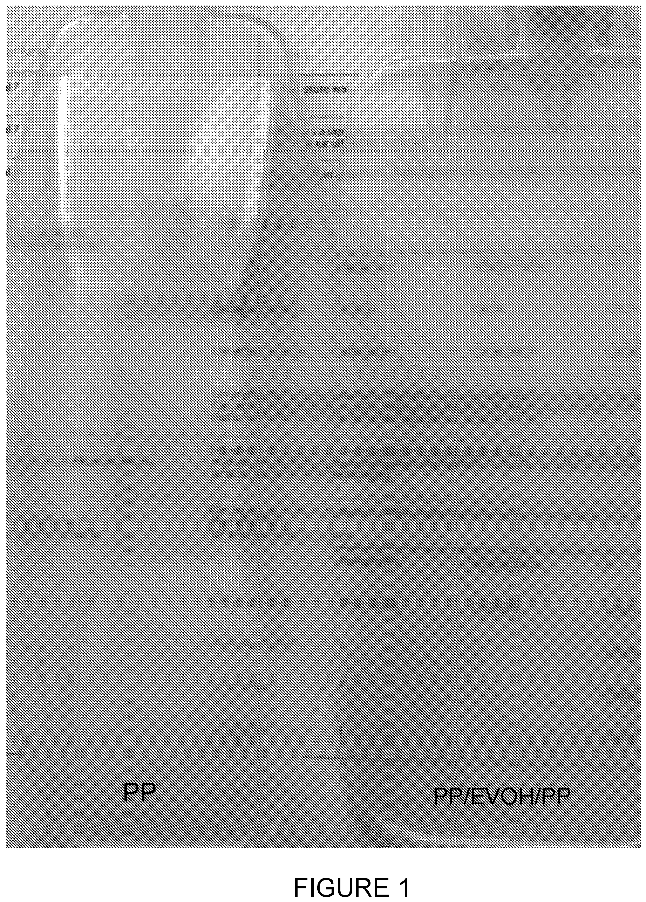

Figure 1 is a photograph of the translucent polypropylene and polypropylene / ethylene vinyl alcohol / polypropylene bags described in Example 2, laid over a black and white image to illustrate contrast, haze, and luminous transmittance.

### DETAILED DESCRIPTION

### Definitions and Use of Terms

Throughout this application, various publications are referenced. The disclosures of these publications in their entireties are hereby incorporated by reference into this application in order to more fully describe the state of the art to which this invention pertains. The references disclosed are also individually and specifically incorporated by reference herein for the material contained in them that is discussed in the sentence in which the reference is relied upon.

As used in the specification and claims, the singular forms a, an, and the include plural references unless the context clearly dictates otherwise. For example, the term "a specification" refers to one or more specifications for use in the presently disclosed methods and systems. "A hydrocarbon" includes mixtures of two or more such hydrocarbons, and the like. The word "or" or like terms as used herein means any one member of a particular list and also includes any combination of members of that list.

As used in this specification and in the claims which follow, the word "comprise" and variations of the word, such as "comprising" and "comprises," means "including but not limited to," and is not intended to exclude, for example, other additives, components, integers or steps. When an element is described as comprising one or a plurality of components, steps or conditions, it will be understood that the element can also be described as "consisting of" or "consisting essentially of" the component, step or condition, or the plurality of components, steps or conditions.

When ranges are expressed herein by specifying alternative upper and lower limits of the range, it will be understood that the endpoints can be combined in any manner that is mathematically feasible. Thus, for example, a range of from 50 or 80 to 100 or 70 can alternatively be expressed as a series of ranges of from 50 to 100, from 50 to 70, and from 80 to 100. When a series of upper bounds and lower bounds are related using the phase "and" or "or", it will be understood that the upper bounds can be unlimited by the lower bounds or combined with the lower bounds, and vice versa. Thus, for example, a range of greater than 40% and/or less than 80% includes ranges of greater than 40%, less than 80%, and greater than 40% but less than 80%.

When an element of a process or thing is defined by reference to one or more examples, components, properties or characteristics, it will be understood that any one or combination of those components, properties or characteristics can also be used to define the subject matter at issue. This might occur, for example, when specific examples of an element are recited in a claim (as in a Markush grouping), or an element is defined by a plurality of characteristics. Thus, for example, if a claimed system comprises element A defined by elements A1, A2 and A3, in combination with element B defined by elements B1, B2 and B3, the invention will also be understood to cover a system defined by element A without element B, a system in which element A is defined by elements A1 and A2 in combination with element B defined by elements B2 and B3, and all other possible permutations.

When used herein the term "about" will compensate for variability allowed for in the pharmaceutical industry and inherent in products in this industry, such as differences in product strength due to manufacturing variation and time-induced product degradation. The term allows for any variation which in the practice of good manufacturing practices would allow the product being evaluated to be considered therapeutically equivalent or bioequivalent in humans to the recited strength of a claimed product.

The phrase "acceptable" as used in connection with compositions of the invention, refers to molecular entities and other ingredients of such compositions that are physiologically tolerable and do not typically produce untoward reactions when administered to a subject (e.g., a mammal such as a human).

When published test methodologies and diagnostic instruments are referred to herein, it will be understood that the test methodology or diagnostic instrument is performed based on the version in effect on October 1, 2020, unless otherwise stated to the contrary herein.

Translucent means admitting or diffusing light so that objects beyond cannot be clearly distinguished. Transparent means having the property of transmitting light without appreciable scattering so that bodies lying beyond are entirely visible. For purposes of this invention, the bags depicted in Figure 1 would be considered translucent because the letters beyond the bag can be distinguished, even though the identity of the letters is difficult to discern.

Within the present invention, "injectable" means suitable to be injected into a patient (human or animal). Typically, the noradrenaline solution of the invention is administered by intravenous or intra-arterial injection. In certain embodiments, there is provided an infusion of a therapeutically active amount of the noradrenaline solution according to the invention.

Within the present invention, the term "antioxidant" shall mean any antioxidant known in the art which is added to a solution of noradrenaline of a pharmaceutically acceptable salt thereof in order to protect noradrenaline from oxidation. Examples of antioxidants are sulfite(s) or ascorbic acid, or the antioxidants disclosed in US2016/0058715A1, see in particular paragraph [0016] thereof, or other antioxidants known in the art. When a formulation is said to lack antioxidants in this document, it will be understood that the formulation could be defined as lacking any antioxidant as that term is traditionally used in the art, as described, for example, in Rowe et al., Handbook of Pharmaceutical Excipients, 6th Edition 2009. Alternatively, it will be understood that the formulation could be defined as lacking any sulfites or lacking any sulfites or ascorbic acid.

Within the present invention, the term "chelating agent(s)" shall mean any chelating agent known in the art which is added to a solution of noradrenaline of a pharmaceutically acceptable salt thereof in order to protect noradrenaline from degradation. Examples include in particular metal ion chelators, such as EDTA, EGTA, DTPA and the like; see e.g. WO2018/140894A1, in particular paragraph [0024] thereof; the tartrate/bitartrate salt of noradrenaline is not considered a chelating agent within the present invention. When a formulation is said to lack chelating agents in this document, it will be understood that the formulation could be defined as lacking any chelating agent as that term is traditionally used in the art, as described, for example, in Rowe et al., Handbook of Pharmaceutical Excipients, 6th Edition 2009. Alternatively, it will be understood that the formulation could be defined as lacking edetic acid or any salt thereof such as disodium edetate (collectively "EDTA").

The concentrations of noradrenaline or a pharmaceutically acceptable salt thereof are indicated as mg or µg calculated as noradrenaline free base, per ml of the total solution, unless indicated otherwise.

The concentration of oxygen is indicated as percent (%) w/w for the headspace, or ppbw for the dissolved oxygen.

### Principal Embodiments

In a first principal embodiment the invention provides a hermetically sealed noradrenaline drug product in an intravenous ready to use translucent or transparent collapsible drip bag having low subvisible particle counts comprising: (a) a headspace consisting essentially of an inert gas and < 2% v/v oxygen; and (b) a liquid solution comprising: (i) noradrenaline or a pharmaceutically acceptable salt thereof at a concentration of from 0.001 to 0.2 mg/ml or from 0.001 to 0.035 mg/ml, (ii) less than 600 ppbw oxygen, (iii) ≤ 25 per mL subvisible particles equal to or greater than 10 µm, and (iv) ≤ 3 per mL subvisible particles equal to or greater than 25 µm; wherein: (i) the concentration of noradrenaline or pharmaceutically acceptable salt is based on the weight of the free base of noradrenaline (ii) the subvisible particle count is determined by the light obscuration particle count test in USP (May 1, 2013); (iii) the oxygen content in the headspace and solution is measured immediately after the product is hermetically sealed or after the oxygen content in the headspace and solution has reached equilibrium (iv) the particle counts are observed immediately after the product is hermetically sealed or after the oxygen content in the headspace and solution has reached equilibrium; (v) the drug product is free of antioxidants and chelating agents.

In a second principal embodiment, the invention provides a hermetically sealed noradrenaline drug product in an intravenous ready to use translucent or transparent collapsible drip bag having low subvisible particle counts comprising: (a) a headspace consisting essentially of an inert gas, ≤ 1.5% v/v oxygen immediately after the bag is hermetically sealed, and ≤ 1.2% v/v after the oxygen in the headspace and solution have reached equilibrium; and (b) an aqueous liquid solution comprising: (i) noradrenaline or a pharmaceutically acceptable salt thereof at a concentration of from 0.001 to 0.2 mg/ml or from 0.001 to 0.035 mg/ml, (ii) a tonicifying effective amount of sodium chloride, (iii) hydrochloric acid in an amount effective to impart a pH of from 3.0 to 4.5, (iv) less than 200 ppbw oxygen immediately after the product has been hermetically sealed, (v) less than 500 ppbw oxygen after the oxygen in the solution and headspace have reached equilibrium, (vi) ≤ 25 per mL subvisible particles equal to or greater than 10 µm, and (vii) ≤ 3 per mL subvisible particles equal to or greater than 25 µm; wherein: (i) the concentration of noradrenaline or pharmaceutically acceptable salt is based on the weight of the free base of noradrenaline; (ii) the subvisible particle count is determined by the light obscuration particle count test in USP (May 1, 2013); (iii) the particle counts are observed immediately after the product is hermetically sealed and after the oxygen content in the headspace and solution has reached equilibrium; and (iv) the drug product is free of chelating agents and antioxidants.

In a third principal embodiment the invention provides a method of making a hermetically sealed noradrenaline drug product in an intravenous ready to use translucent or transparent collapsible drip bag having low subvisible particle counts comprising: (a) dissolving noradrenaline or a pharmaceutically acceptable salt thereof in water to produce a noradrenaline liquid solution at a concentration of from 0.001 to 0.2 or from 0.001 to 0.035 mg/ml; (b) distributing the noradrenaline solution and an inert gas into the bag, such that the percentage of the bag's volume occupied by the headspace is from 0 to 25% or from 2 to 15%; and (c) hermetically sealing the bag; wherein: (i) the oxygen concentration in the solution is less than 200 ppbw immediately after sealing the container and less than 500 ppbw after the oxygen content in the headspace and solution have reached equilibrium; (ii) the oxygen concentration in the headspace is less than 1.5 % immediately after sealing the container and less than 1.2% after the oxygen content in the headspace and solution have reached equilibrium; (iii) the drug product omits chelating agents, preservatives, and antioxidants; and (iv) when at equilibrium with the headspace, the oxygen content in the solution is less than 500 ppbw.

### Subembodiments

Various parameters can be used to further define the drug products and methods of manufacture of the current invention, as described in greater detail below. It will be understood that each of the subembodiments described below can be used to further define any of the principal embodiments, and that they can be combined in any combination to create new embodiments that are logically possible.

The interior volume of the bags of the current invention preferably ranges from 75 to 550 ml or from 100 to 350 ml or from 250 to 325 ml. A particularly preferred bag has a nominal volume of 250 ml, and an absolute volume of from 260 to 300 ml. In some subembodiments the percentage of the bag's volume occupied by the headspace is from 0 to 25%, with the remainder of the bag's volume occupied by the drug solution. In other subembodiments the percentage of the bag's volume occupied by the headspace is from 2 to 15%, with the remainder occupied by the drug solution. The headspace preferably consists essentially of an inert gas and trace amounts of oxygen. Preferred inert gases are selected from argon and nitrogen and combinations thereof, with nitrogen being particularly preferred.

The drug product also can be defined by the concentration of oxygen in the headspace at various timepoints. Thus, in some subembodiments, immediately after the drug product is hermetically sealed, the oxygen content in the headspace is less than 2.0% v/v, 1.5% v/v, 1.2% v/v, 1.1% v/v, or 1.0% v/v. Alternatively or in addition, the oxygen content can be defined after the solution and headspace oxygen concentrations reach equilibrium. Thus, the oxygen content in the headspace at equilibrium can be less than 2.0% v/v, 1.5% v/v, 1.2% v/v, 1.1% v/v, or 1.0% vivo

According to a further embodiment of the invention, the oxygen concentration in the headspace immediately after sealing the container is within a certain low range of about 0.2 to about 1.0 percent v/v, preferably in the range of about 0.3 to about 0.95 percent v/v, in particular about 0.5 to about 0.95 percent v/v.

In like manner, the drug product can be defined based on the concentration of oxygen in the drug solution at various timepoints. These concentrations can be in addition to or alternatives to the concentration of oxygen in the headspace. Thus, in various subembodiments, immediately after the container is sealed, the concentration of oxygen in the drug solution preferably will not exceed 200, 175, 150, 125, 100 , 75, or even 50 ppbw. Alternatively or in addition, the oxygen content in the solution at equilibrium preferably will not exceed 600, 550, 500, 450, 400, 350, 300, or 250 ppbw.

The drug product also can be defined based on minimum oxygen concentrations in the headspace and solution. Thus, in any of the embodiments of the current invention, immediately after the product is hermetically sealed, the oxygen content in the headspace can be greater than 0.3%, 0.4%, 0.5%, 0.6%, 0.7%, 0.8%, 0.9%, 1%, or 1.1%. Alternatively or in addition, immediately after the product is hermetically sealed, the oxygen content in the solution can be greater than 50 ppbw, 60 ppbw, 70 ppbw, or 80 ppbw. Alternatively or in addition, after the oxygen content in the headspace and solution have reached equilibrium, the oxygen content in the headspace can be greater than 0.5%, 0.6%, 0.7%, 0.8%, or 0.9%. Alternatively or in addition, after the oxygen content in the headspace and solution have reached equilibrium, the oxygen content in the solution can be greater than 250 ppbw, 275 ppbw, 300 ppbw, 325 ppbw, or 350 ppbw.

Various methods can be used to determine the oxygen content in the headspace and solution at these various time points. One non-invasive method is Frequency Modulation Spectroscopy (FMS), where light from a near-infrared semiconductor laser is tuned to match the internal vibrational frequency of the oxygen molecule in the container, although other methods exist including electrochemical methods. An invasive draw, where the container is physically breached and a sample is taken from the headspace and/or solution is another way. Another way is to calculate the content based on other oxygen values. Thus, for example, following the procedure in Example 3, it is possible to calculate the concentration of the oxygen in the headspace immediately after the bag is sealed knowing the oxygen content in the solution immediately after the bag is filled, the oxygen content in the solution or headspace at equilibrium, the volume of the headspace, and the total volume of the bag. Still another way is to base the concentration on another time point, when there is no oxygen added to the system. For example, the oxygen concentration in the solution after the container is sealed will be the same as the concentration of oxygen in the solution after the oxygen purge, barring infiltration of oxygen into the system, or a subsequent oxygen purge.

At all time points during the shelf life of the product, the subvisible particle concentrations will never exceed the following minimum values: ≤ 25 per mL subvisible particles equal to or greater than 10 µm, and ≤ 3 per mL subvisible particles equal to or greater than 25 µm. Thus, these subvisible particle concentration limits will never be violated either immediately after the bags are hermetically sealed or after the headspace and solution concentrations of oxygen have reached equilibrium.

In preferred embodiments, immediately after the oxygen content in the headspace and solution have reached equilibrium, the subvisible particles equal to or greater than 10 µm will be less than or equal to 15 or 10 per mL. Alternatively or in addition, subvisible particles equal to or greater than 25 µm will be less than or equal to 2 or 1 per mL.

In particularly preferred embodiments, the solution and/or headspace will maintain these subvisible particle counts for one or even two years when stored at 25 °C protected from light. The drug product will preferably experience an increase in the ≥ 10 µm subvisible particle count of no more than 50%, 100%, 200%, 300%, 400%, or 500% for 12 months when stored at 25 °C and 60% relative humidity protected from light. Alternatively or in addition, the drug product will preferably experience an increase in the ≥ 25 µm subvisible particle count of no more than 50%, 100%, 200%, 300%, 400%, or 500% for 12 months when stored at 25 °C and 60% relative humidity protected from light.

The drug product can also be defined based on various formulation parameters. The noradrenaline, for example, can be present as any pharmaceutically acceptable salt. Particularly preferred salts are the hydrochloride salt and the bitartrate salt, with the bitartrate salt most preferred.

The drug product can also be defined based on its stability, and the presence of impurities (i.e. by-products from the manufacturing process of noradrenaline, degradants of noradrenaline, and contaminants, but excluding residual solvents) in the solution. In preferred embodiments, the solution comprises no more than 1%, 0.5%, 0.2%, or even 0.1% (w/w) impurities and degradants of noradrenaline.

The noradrenaline can be present in various concentrations, always calculated based on the weight of the free base. In some embodiments, the concentration of noradrenaline is from 0.001 to 0.2 mg/ml. In other embodiments the concentration of the noradrenaline is from 0.001 to 0.035 mg/ml. In still further embodiments the solution comprises from 0.01 to 0.2 mg/ml noradrenaline bitartrate based on the weight of the free base. In still other embodiments the solution comprises approximately 0.016, 0.032, or 0.128 mg/ml noradrenaline bitartrate based on the weight of the free base.

The solution also preferably includes a pharmaceutically acceptable tonicity agent and a pharmaceutically acceptable acid capable of adjusting the pH of the solution to 3.0 to 4.5. Suitable tonicity agents can be found in the United States Pharmacopoeia and include, for example, dextrose, glycerin, mannitol, potassium chloride and sodium chloride, with sodium chloride being most preferred. The tonicity agent is preferably present in concentrations adequate to render the solution isotonic, i.e. about 290 mOsm/kg, or substantially isotonic, i.e. from 250 to 350 mOsm/kg.

Suitable acids include, for example, Acetic Acid, glacial, USP, Acetic Acid, NF, Citric Acid, anhydrous USP, Citric Acid, monohydrate USP, Fumaric Acid, NF, Hydrochloric Acid, diluted, NF, Hydrochloric Acid, NF, Lactic Acid, USP, Malic Acid, NF, Nitric Acid, NF, Phosphoric Acid, NF, Phosphoric Acid, diluted, NF, Propionic Acid, NF, Sodium phosphate monobasic, NF, Sulfuric Acid, NF, and Tartaric Acid, NF, with hydrochloric acid being most preferred. The acid is added in a quantity sufficient to impart a pH to the solution of from 3.0 to 4.5, preferably from 3.0 to 3.8 or from 3.1 to 3.6.

The drug product can also be defined in terms of the bag in which it is housed. Thus, for example, while the bag can assume any configuration that permits filling, sealing, and subsequent access during use, in preferred embodiments the bag comprises two opposed walls sealed around their peripheries.

The walls of the bag are preferably constructed of single ply polypropylene or multi-ply product incorporating a polypropylene inner ply and a second ply made from an ethylene vinyl alcohol copolymer. In a preferred embodiment, the bag wall is a multi-ply product comprising two polypropylene layers and an ethylene vinyl alcohol layer sandwiched between the two polypropylene layers.

A preferred polypropylene is a translucent single ply random copolymer polypropylene such as the polypropylene ply based on a phthalate-free catalyst marketed by Total Research and Technology Feluy (Feluy, Belgium) under the trade name Polypropylene Aceso^{®} PPM R020 S01. A particularly preferred material is the single ply random copolymer polypropylene, having substantially the properties described in Table 2a.

A preferred material for the second ply is an ethylene vinyl alcohol copolymer having the structure:

-[CH₂-CH₂]ₙ-[CH(OH)-CH₂]ₘ- or -[CH₂-CH₂]ₙ-[CH₂-CH(OH)]ₘ-

comprising less than 35 or 30 or mol % ethylene, preferably about 27 mol % ethylene. A particularly preferred material is an ethylene vinyl alcohol copolymer comprising 27 mol % ethylene, having substantially the physical properties reported in Table 2b.

In other embodiments the material used for the bag wall is defined based on its optical properties. In one particular embodiment the bag exhibits the optical properties of one or both of the bags depicted in Figure 1. In another particular embodiment, the bag exhibits the optical properties of the material described in Table 2a and/or 2b. Optionally, the optical properties can be determined by the methods described in ASTM D 1003-61, and the plastic will have substantially the same haze and luminous transmittance values as one or both of the translucent polypropylene depicted in Figure 1 or described in Table 2a and/or Table 2b. For purposes of this invention, it will be understood that the bags depicted in Figure 1 have substantially equivalent optical properties.

In still further embodiments, the bag walls are characterized by an oxygen transmission rate of less than 0.2 or 0.15 or 0.12 cm³.20µm/m².day.atm when measured according to ISO 14663-2 Annex C.

Still further embodiments define the material used to make the bag walls in terms of their ability to maintain subvisible particle counts over the life of the product. Thus, in still another embodiment, the walls are constructed of means for maintaining the subvisible particle counts for one year when stored at 25 °C and 60 percent relative humidity protected from light at the following levels:
- ≤ 25 per mL subvisible particles equal to or greater than 10 µm, and
- ≤ 3 per mL subvisible particles equal to or greater than 25 µm.

In particularly preferred embodiments, the "means for maintaining" will maintain these subvisible particle counts for one or even two years when stored at 25 °C protected from light. The means for maintaining will preferably prevent an increase in the ≥ 10 µm subvisible particle count of no more than 50%, 100%, 200%, 300%, 400%, or 500% for 12 months when stored at 25 °C and 60% relative humidity protected from light. Alternatively or in addition, the means for maintaining will preferably experience an increase in the ≥ 25 µm subvisible particle count of no more than 50%, 100%, 200%, 300%, 400%, or 500% for 12 months when stored at 25 °C and 60% relative humidity protected from light.

The structure for this "means for maintaining" corresponds to a polypropylene ply displaying the haze and luminous transmittance of the single ply polypropylene bag in Figure 1, as further described in Table 2a. The PP/EVOH/PP bag depicted in Figure 2 performs an equivalent function, as exhibited by the subvisible particle formation data in Example 2.

Still further subembodiments relate specifically to the method of manufacturing the bags of the current invention. Thus, in any of the methods of the current invention, the manufacturing process is undertaken in the presence of light without any extra precautions taken against the presence of light. Still further embodiments relate to the drug product manufactured by the method of any of the methods of the current invention.

The process for manufacturing the drug product of the current invention will typically be performed as described in WO2015/128418 A1. Thus, the water used to prepare the solution is typically degassed or deaerated, distilled, sterile, pyrogen-free water for pharmaceutical use. According to some embodiments, the deoxygenated or degassed water is obtained by blowing or bubbling an inert gas current.

Noradrenaline and optionally any excipient(s) are dissolved in the degassed water. According to one preferred embodiment, dissolution can be carried out within a suitable inert container, in which air or oxygen have been removed by passage of inert gas. According to a further preferred embodiment, during dissolution of the noradrenaline an inert gas can be blown into the container or tank of the solution, to remove any residual oxygen. Upon completion of the deaerating step, the noradrenaline solution obtained will typically have has a residual oxygen content (dissolved oxygen) equal to or lower than 300 ppbw, 200 ppbw, 150 ppbw, preferably equal to or lower than 100 ppbw.

The noradrenaline solution is also distributed into suitable containers. The containers may be depyrogenated, optionally in the presence of inert gas. Particular care is taken to minimize or avoid oxygen or air entering the container during the distributing (filling) step. As it was found that both the oxygen content in the headspace as well as the dissolved oxygen content in the solution have to be tightly controlled within the limits disclosed herein, the parameters of the distributing devices and environments for all steps are optimized and verified to constantly achieve the desired oxygen levels. Extra measures may comprise increasing the flow and direct insertion of an inert gas into the container during the distribution step, e.g. by using a coaxial needle device distributing both the noradrenaline solution and an inert gas or inert gas mixture from two coaxial flow channels into the container, or application of a drop of liquid nitrogen into the container after completely distributing (filling) the noradrenaline solution into the container, but before the container is sealed. It is also possible to place the device used for the distribution of the solution into the container in a reduced oxygen or substantially oxygen free environment for further minimizing the amount of oxygen entering the container, and to use closed filling lines excluding the ingress of oxygen.

The drug product is sterilized by known means. Such known means in the art comprise e.g. sterile filtration, heat treatment and/or irradiation, in particular sterile filtration and/or heat treatment, with sterile filtration most preferred. The heat treatment, if performed, may be accomplished typically at temperatures above 100°C, for a time suitable for sterilization, for example equal to or greater than 15 minutes. Preferably, the heat treatment can be a heat sterilization for 15 minutes at 121 °C, in particular in an autoclave.

The sterile filtration, if performed, may be done by passing the solution containing noradrenaline through a filter of the type for sterilization, as known in the art. Passage of the noradrenaline solution through the filter can be sped up by blowing a current of inert gas which acts as a carrier. Suitable filters are those used in the pharmaceutical technology for preparation of sterile injectable solutions.

The drug product is also hermetically sealed. Sealing can be done in any conventional way, taking care that during the sealing step oxygen (or air) is kept out of the container, e.g. by a flow of inert gas (such as through a coaxial needle), applying a drop of liquid nitrogen into the container before sealing, or applying vacuum or inert gas atmosphere. The device used for sealing the container can be placed in a reduced oxygen or substantially oxygen free environment for further minimizing the amount of oxygen entering the container during the sealing step d).

Preferably, the steps of the process of producing a noradrenaline solution as disclosed herein are carried out in sterile environments in order to avoid bacterial contamination of the noradrenaline solution.

According to a further aspect of the present invention, the noradrenaline solution or drug product of the invention as described herein is used as a parenteral dosage form, in particular a ready-to-administer parenteral dosage form, preferably in the treatment of cardiac circulatory collapse, in states of shock associated with low peripheral resistances or to restore and/or keep physiological pressure levels.

### EXAMPLES

In the following examples, efforts have been made to ensure accuracy with respect to numbers (e.g., amounts, temperature, etc.) but some errors and deviations should be accounted for. The following examples are put forth so as to provide those of ordinary skill in the art with a complete disclosure and description of how the methods claimed herein are made and evaluated, and are intended to be purely exemplary of the invention and are not intended to limit the scope of what the inventors regard as their invention.

### Example 1: Effect of Oxygen Concentrations on Oxidation By-Products and Subvisible Particle Formation

A study was undertaken to evaluate the effect of oxygen content in the headspace of 50 ml noradrenaline bitartrate vials on product stability, analyzing for noradrenaline bitartrate (NA) concentration (assay %) oxygen (% v/v), subvisible particle counts ≥ 10 µm, subvisible particle counts ≥ 25 µm, and arterenone concentration (mg/ml) at different time points. Particle counts were measured using the light obscuration particle count test prescribed by USP chapter 788 (Official as of 1 May 2013). All testing was undertaken according to stability methods prescribed by the International Conference on Harmonization, ICH Q1A (R2) (effective 1 August 2003).

Production batches of 50 ml glass vials were manufactured at noradrenaline concentrations ranging from 0.06 mg/ml to 0.2 mg/ml. Multiple production batches were evaluated for this Example; between 16 and 160 samples were withdrawn from each production batch, and evaluated for oxygen content in the headspace shortly after filling. The oxygen contents of the samples were then summed and averaged to give an average oxygen content for each batch. Vials were filled in an inert nitrogen atmosphere to an average solution volume of 52.5 ml, with an average headspace volume of 3.5 ml (i.e. 6.25%). The solution itself was purged with nitrogen prior to ingredient dissolution and filling into the vials, and constantly kept under strict exclusion of oxygen/air such that oxygen content in the final solution was consistently maintained below 50 ppbw, including the use of a coaxial needle for filling the vials with the noradrenaline solution and a stream of nitrogen at the same time.

Product was manufactured aseptically, without a high temperature terminal sterilization, and without protection from light. 50 ml vials used for this Example were Type I clear colorless glass vials closed with a bromo-butyl stopper and an aluminum flip-off cap. The formulations consisted of noradrenaline bitartrate, sodium chloride, hydrochloric acid 1 N (for pH-adjustment), and water for injection. The final pH of the solution was 3.0-4.5. The final Osmolarity was 250 - 350 mOsm/kg.

Results for room temperature stability testing (25 °C, 60% RH) and accelerated stability testing (40 °C, 75% RH) at the 24-month and 6-month time points are presented below in Tables 1a and 1b. The results are divided with high O₂ concentration headspace batches (> 1%) below the line and low O₂ concentration headspace batches (< 1%) above the line. Equilibrium solution concentrations were calculated according to the method of Example 3, assuming an initial oxygen concentration in solution of 50 ppbw.

For perspective, it should be noted that the USP chapter 788 allows for 6000 particles ≥ 10 µm and 600 particles ≥ 25 µm when measured according to the light obscuration particle count test in containers less than 100 ml.

**Table 1a (25 °C, 60% RH, 24 months)**

| NA Conc. (mg/ml) | Headspace O₂* | Particles** ≥ 10 µm | Particles** ≥ 25µm | Arterenone |
|---|---|---|---|---|
| 0.06 | 0.41 | 1313 | 33 | 0.1 |
| 0.1 | 0.49 | 613 | 2 | 0.06 |
| 0.12 | 0.58 | 1055 | 10 | 0.06 |
| 0.2 | 0.47 | 1880 | 38 | 0.05 |
| 0.2 | 0.39 | 2472 | 32 | |
| 0.1 | 1.77 | 7228 | 118 | 0.06 |
| 0.1 | 1.26 | 7302 | 216 | 0.06 |
| 0.2 | 1.21 | 6626 | 110 | 0.06 |
| 0.2 | 1.50 | 13845 | 455 | 0.06 |
| 0.2 | 1.59 | 8478 | 356 | 0.07 |

| | | | | |
|---|---|---|---|---|
| *Immediately after vial is sealed. **Particle counts are particles per 50 ml vial at T₀ | | | | |

**Table 1b (40 °C. 75% RH, 6 months)**

| NA Conc. (mg/ml) | Headspace O₂* | Particles** ≥ 10 µm | Particles** ≥ 25 µm | Arterenone | O₂ in solution at equilibrium (25°C)*** |
|---|---|---|---|---|---|
| 0.06 | 0.41 | 1145 | 13 | 0.11 | 142 |
| 0.1 | 0.49 | 386 | 6 | 0.09 | 166 |
| 0.1 | 0.59 | 532 | 5 | 0.12 | 197 |
| 0.1 | 0.72 | 992 | 48 | 0.07 | 237 |
| 0.12 | 0.52 | 693 | 19 | 0.07 | 176 |
| 0.12 | 0.58 | 750 | 24 | 0.09 | 194 |
| 0.2 | 0.64 | 832 | 22 | 0.04 | 213 |
| 0.2 | 0.39 | 1417 | 19 | 0.06 | 136 |
| 0.1 | 1.42 | 5090 | 124 | 0.07 | 452 |
| 0.1 | 1.26 | 5700 | 297 | 0.04 | 403 |
| 0.2 | 1.21 | 5157 | 207 | 0.03 | 388 |
| 0.2 | 1.50 | 4567 | 237 | 0.07 | 477 |
| 0.2 | 1.59 | 7887 | 397 | 0.1 | 504 |

| | | | | | |
|---|---|---|---|---|---|
| *Immediately after vial is sealed. **Particle counts are particles per 50 ml vial at T₀ ***T_{eq} = 25 °C; calculated by the method of Example 3 | | | | | |

Several observations can be made based on the data reported in Tables 1a and 1b.

First, based on the arterenone data, oxidation was not affected by whether the batch had a high oxygen concentration in the headspace or a low oxygen concentration in the headspace. However, subvisible particle formation was greatly affected by the concentration of oxygen in the headspace, and improved at lower oxygen concentrations. In fact, at oxygen headspace concentrations greater than 1% during product manufacture, most of the products approached the USP limits on subvisible particles and several products exceeded the USP limit on subvisible particles at the time points reported. This discovery was surprising, and has tremendous practical significance because it avoids the need to investigate and eliminate other potential sources of subvisible particle formation.

Second, the products above the line were not at significant risk of violating USP subvisible particles limits at either time point under either testing condition, whereas several products below the line were. The equilibrium oxygen concentrations in solution, when calculated according to the method of Example 3, were consistently less than 340 ppbw for products above the line, and consistently greater than 340 ppbw for products below the line.

Based on these observations, a further analysis was undertaken to understand the kinetics of subvisible particle formation above and below the line for target oxygen concentrations in solution. As can be seen from Table 1c below, the subvisible particles did not meaningfully increase in the first three months of storage, if an oxygen content of less than 1% v/v oxygen in the headspace was used. In fact, the number of subvisible particles significantly decreased when comparing the counts immediately after sealing the container, and after 3 months storage, if using a headspace with an oxygen content of less than 1% v/v immediately after sealing the vials. Some of the smallest subvisible particles even appeared to re-dissolve.

**Table 1c (25 °C. 60% RH storage)**

| NA Conc. (mg/ml) | Headspace O₂* | Particles ≥ 10 µm at T₃ₘₒ as % of particles ≥ 10 µm at T₀ |
|---|---|---|
| 0.06 | 0.41 | 5% |
| 0.1 | 0.49 | 39% |
| 0.12 | 0.58 | 2% |
| 0.2 | 0.47 | 18% |
| 0.2 | 0.39 | 10% |
| 0.1 | 1.77 | 648 % |
| 0.1 | 1.26 | 475% |
| 0.2 | 1.21 | 1033% |
| 0.2 | 1.50 | 1681% |
| 0.2 | 1.59 | 1060% |

| | | |
|---|---|---|
| *Immediately after the vial is sealed. | | |

### Example 2: Effect of Packaging Material and Oxygen Permeability on Oxidation By-Products and Subvisible Particle Formation

A second stability study was undertaken to evaluate the effect of packaging material on 250 ml ready to use (RTU) collapsible pouches of noradrenaline bitartrate on product stability, analyzing for noradrenaline bitartrate (NA) concentration (assay %), subvisible particle counts ≥ 10 µm, subvisible particle counts ≥ 25 µm, and arterenone concentration at different time points under different storage conditions. Particle counts were measured using the light obscuration particle count test prescribed in USP chapter 788 (Official as of 1 May 2013). All testing was undertaken according to stability methods prescribed by the International Conference on Harmonization, ICH Q1A (R2) (effective 1 August 2003).

Pilot batches of noradrenaline in 250 ml pouches were manufactured at concentrations ranging from 0.016 mg/ml to 0.128 mg/ml. Pouches were filled in an inert nitrogen atmosphere to a solution volume on average of 262 ml, with an average headspace volume of 18 ml (i.e. 6.4%). The solution itself was purged with nitrogen prior to ingredients dissolution and filling into the pouches, such that oxygen content in the final solution was consistently maintained below 100 ppbw.

Product was manufactured aseptically, without high temperature terminal sterilization or protection from light. The 250 ml pouches used for this Example were manufactured using either a single ply random copolymer polypropylene based on a phthalate-free catalyst marketed by Total Research and Technology Feluy (Feluy, Belgium), under the trade name Polypropylene Aceso^{®} PPM R020 S01, or a three ply polypropylene/EVOH copolymer/polypropylene bag wall with the EVOH copolymer being a 27 mol% Ethylene-Vinyl Alcohol Copolymer marketed by Kuraray America, Inc. (Houston, Texas) under the trade name EVAL^{™} L171B. The formulations consisted of noradrenaline bitartrate, sodium chloride, hydrochloric acid 1 N (for pH-adjustment), and water for injection. The final pH of the solution was 3.0-4.5. The final Osmolarity was 250 - 350 mOsm/kg.

Polypropylene Aceso^{®} PPM R020 S01 and EVAL^{™} L171B polymers are characterized by the physical properties in Tables 2a and 2b:

**Table 2a (Polypropylene Aceso^{®} PPM R020 S01)**

| | | | **Method** | **Unit** | **Typical Value** |
|---|---|---|---|---|---|
| **Rheological Properties** | | | | | |
| | Melt Flow Index 230 °C/2.16 kg | | ISO 1133 | g/10 min | 1.8 |

| **Mechanical Properties** | | | | | |
|---|---|---|---|---|---|
| | Tensile Strength at Yield | | ISO 527-2 | MPa | 26 |
| | Elongation at Yield | | ISO 527-2 | % | 10 |
| | Tensile Modulus | | ISO 527-2 | MPa | 1000 |
| | Flexural Modulus | | ISO 178 | MPa | 900 |
| | Izod Impact Strength (notched) at 23°C | | ISO 180 | kJ/m² | 6 |
| | Charpy impact strength (notched) at 23°C | | ISO 179 | kJ/m² | 8 |
| | Rockwell Hardness - R-scale | | ISO 2039-2 | | 82 |

| **Thermal Properties** | | | | | |
|---|---|---|---|---|---|
| | Melting Point | | ISO 3146 | °C | 149 |
| | Vicat Softening Point | | ISO 306 | °C | |
| | | 50N - 50°C per hour | | | 67 |
| | | 10N - 50°C per hour | | | 130 |

| **Other Physical Properties** | | | | | |
|---|---|---|---|---|---|
| | Density | | ISO 1183 | g/cm³ | 0.902 |
| | Bulk Density | | ISO 1183 | g/cm³ | 0.525 |

**Table 2b (EVAL^{™} L171B)**

| **Typical Properties** | **Unit** | **Test Method** | **Metric/(English)** |
|---|---|---|---|
| MFR | g/10min | ISO1133 (210°C) | 4.0 |
| Density | 10³ kg/m³ | ISO1183-3 | 1.21 |

| **Thermal Properties** | **Unit** | **Test Method** | **Metric/(English)** |
|---|---|---|---|
| Melting Temp | °C (°F) | ISO 11357 | 190 (374) |
| Crystallization Temp | °C (°F) | ISO 11357 | 164 (327) |
| Glass Transition Point | °C (°F) | ISO 11357 | 63 (145) |
| Vicat Softening Point | °C (°F) | ISO 306 | 181 (358) |

| **Mechanical Properties** | **Unit** | **Test Method** | **Metric/(English)** |
|---|---|---|---|
| Tensile stress at break | MPa (psi) | ISO 527 | 41 (5,900) |
| Elongation at break | % | ISO 527 | 12 |
| Young's Modulus | GPa (psi) | ISO 527 | 4.9 (710,000) |
| Flexural Modulus | GPa (psi) | ISO 178 | 4.7 (680,000) |
| Charpy Impact Strength | kJ/m² (ft.lbf/in²) | ISO 179-1 | 6 (3.0) |
| Rockwell Hardness | HRM | ISO 2039-2 | 97 |

| **Barrier Properties (cast film)** | **Unit** | **Test Method** | **Metric/(English)** |
|---|---|---|---|
| Oxygen Transmission Rate | cm³.20pm/m².day.atm (cm³.mil/100in².day.atm) | ISO 14663-2 | 0.1 (0.005) |

The PP and PP/EVOH/PP layers in both bags were translucent without any pigments or opacifying agents or opaque plies layered thereon. Photographs of the PP and PP/EVOH/PP bags are presented in Figure 1.

Results for accelerated stability testing (40 °C, 75% RH) at T₀ and at the 6-month stability time point are presented below in Tables 2c and 2d.

For perspective, it should be noted that the USP chapter 788 allows for 25 particles ≥ 10 µm per ml and 3 particles ≥ 25 µm per ml when measured according to the light obscuration particle count test in containers greater than 100 ml.

**Table 2c (40 °C. 75% RH. T₀)**

| Package NA conc. | PP 16 µg/ml | PP/EVOH/PP 16 µg/ml | PP 32 µg/ml | PP/EVOH/PP 32 µg/ml | PP 128 µg/ml | PP/EVOH/PP 128 µg/ml |
|---|---|---|---|---|---|---|
| Arterenone assay | 0.01 | 0.01 | 0.01 | 0.01 | 0.01 | 0.01 |
| Unknown impurities | 0.09 | 0.10 | 0.10 | 0.09 | 0.10 | 0.09 |
| Particles ≥ 10 µm* | 2 | 4 | 1 | 1 | 1 | 1 |
| Particles ≥ 25 µm* | 1 | 2 | 1 | 1 | 1 | 1 |

| | | | | | | |
|---|---|---|---|---|---|---|
| *particle counts are particles per milliliter | | | | | | |

**Table 2d (40 °C. 75% RH, 6 months)**

| Package NA conc. | PP 16 µg/ml | PP/EVOH/PP 16 µg/ml | PP 32 µg/ml | PP/EVOH/PP 32 µg/ml | PP 128 µg/ml | PP/EVOH/PP 128 µg/ml |
|---|---|---|---|---|---|---|
| Arterenone assay | 0.25 | 0.18 | 0.28 | 0.18 | 0.09 | 0.09 |
| Unknown impurities | 2.75 | 1.68 | 2.58 | 1.52 | 1.53 | 0.84 |
| Particles ≥ 10 µm* | 1 | 8 | 7 | 1 | 13 | 13 |
| Particles ≥ 25 µm* | 0 | 1 | 1 | 0 | 1 | 1 |

| | | | | | | |
|---|---|---|---|---|---|---|
| *particle counts are particles per milliliter | | | | | | |

Oxygen content in the headspace and the solution are provided in Table 2d. The oxygen content was determined in the solution at equilibrium and in the solution and headspace immediately after the bag was sealed. The oxygen content in the headspace at T₀ was calculated according to the method of Example 3.

**Table 2e**

| Package NA conc. | PP 16 µg/ml | PP/EVOH/PP 16 µg/ml | PP 32 µg/ml | PP/EVOH/PP 32 µg/ml | PP 128 µg/ml | PP/EVOH/PP 128 µg/ml |
|---|---|---|---|---|---|---|
| Headspace O₂ | 1.28 | 1.26 | 1.33 | 1.30 | 1.27 | 1.28 |
| Solution O₂ | 99 | 99 | 90 | 90 | 97 | 97 |
| Solution O₂ Equilibrium | 430 | 424 | 443 | 434 | 427 | 430 |

Several conclusions can be drawn from this data.

First, even though the equilibrium concentrations of oxygen in solution were well above the 340 ppbw cutoff established in Example 1 for 50 ml glass vials, the particle counts did not approach the subvisible particle count limits established by the USP for the polypropylene and PP/EVOH/PP RTU bags at any of the dilute concentrations tested. This result clearly suggests some degree of protection afforded by the polypropylene layer.

Photostability testing of Applicant's marketed 1 mg/ml noradrenaline ampules support a similar conclusion. Glass ampules tested according to ICH Q1B "Photostability testing of new active substances and Medicinal products" show no difference in stability when exposed to light or protected from light by an aluminum foil over-wrap.

Second, the PP/EVOH/PP pouches produced less oxidative degradation than the PP pouches. However, both pouches performed equally well in terms of subvisible particles. This data is consistent with data in Example 1 showing that subvisible particle formation in dilute solutions, below a particular threshold for oxygen content, is not a function of oxidative degradation.

Third, even though the RTU bags were manufactured and filled without protection from light, and packaged in translucent plastic pouches, both types of plastic materials maintained excellent stability over time particularly in terms of subvisible particles. This is contrary to the teachings of Dinnequin (US 2005/0070613 A1), which reported significant degradation of noradrenaline when manufactured in the presence of light.

Fourth, a slight trend toward greater subvisible particle concentrations was observed with higher noradrenaline concentrations. This result is similar to the results reported in Example 1, and further supports the conclusion that subvisible particle formation is not an oxidation phenomenon, inasmuch as oxidation as a percentage of available drug product is more likely in dilute solutions.

These observations support the conclusion that the optical properties of the polypropylene lining are protecting the drug product from subvisible particle formation.

### Example 3: Calculation of Oxygen Concentrations in Headspace and in the Solution Volume at Equilibrium

The following procedure can be used to determine the concentrations of oxygen in solution and in the headspace in a sealed container after the oxygen between these two phases has been allowed to equilibrate. This method can be used regardless of the container selected or the volume inside the container, as long as the oxygen in the headspace is initially greater than the oxygen in the headspace at equilibrium.

This example assumes a liquid volume of 50 mL and a gaseous headspace volume of 6 mL, a dimensionless Henry's Law constant (K_{H}) of 3.2×10⁻², an initial amount of oxygen in water of 5 µg, and an initial amount of oxygen in the gaseous headspace of 85 µg. Dimensionless Henry's constants K for oxygen, nitrogen and argon in water are 3.2×10⁻², 1.5×10⁻², and 3.4×10⁻², respectively. The equilibrium equation can be depicted as follows:

Oxygen (g) ↔ Oxygen (aq)

Assuming no or negligible chemical or biological loss, equilibrium laws indicate an x increase for the right-hand side of the equation compensating an identical decrease for the lefthand part, all governed by equilibrium constant: K = [[mol(aq) + x]/V(aq)] / [[mol(g) - x]/V(g)] Solving for x, and adding it to the initial contribution in water, one finds a final dissolved concentration of 381 micrograms/L.

## Claims

1. A hermetically sealed noradrenaline drug product in an intravenous ready to use translucent or transparent collapsible drip bag having low subvisible particle counts comprising:
a) a headspace consisting essentially of an inert gas and < 2% v/v oxygen; and
b) a liquid solution comprising:
i) noradrenaline or a pharmaceutically acceptable salt thereof at a concentration of from 0.001 to 0.2 mg/ml or from 0.001 to 0.035 mg/ml,
ii) less than 600 ppbw oxygen,
iii) ≤ 25 per mL subvisible particles equal to or greater than 10 µm, and
iv) ≤ 3 per mL subvisible particles equal to or greater than 25 µm;
wherein:
i) the concentration of noradrenaline or pharmaceutically acceptable salt is based on the weight of the free base of noradrenaline;
ii) the subvisible particle count is determined by the light obscuration particle count test in USP <788> (May 1, 2013);
iii) the oxygen content in the headspace and solution is measured immediately after the product is hermetically sealed or after the oxygen content in the headspace and solution has reached equilibrium;
iv) the particle counts are observed immediately after the product is hermetically sealed or after the oxygen content in the headspace and solution has reached equilibrium;
v) the drug product is free of antioxidants and chelating agents.

2. The drug product of claim 1, wherein the volume of the bag is from 75 to 550 ml or from 100 to 350 ml or from 250 to 325 ml, and the percentage of the bag's volume occupied by the headspace is from 0 to 25% or from 2 to 15%.

3. The drug product of claim 1 or 2, wherein, immediately after the product is hermetically sealed,
a) the oxygen content in the headspace is less than 1.5% v/v or 1.2% v/v; and
b) the oxygen content in the solution is less than 200 ppbw or 120 ppbw.

4. The drug product of any of the preceding claims wherein, immediately after the product is hermetically sealed, the solution comprises ≤ 10 per mL subvisible particles equal to or greater than 10 µm, and ≤ 2 per mL subvisible particles equal to or greater than 25 µm.

5. The drug product of any of the preceding claims wherein, after the oxygen content in the headspace and solution have reached equilibrium,
a) the oxygen content in the headspace is less than 1.5% v/v; and
b) the oxygen content in the solution is less than 500 ppbw.

6. The drug product of any of the preceding claims wherein, immediately after the oxygen content in the headspace and solution have reached equilibrium, the solution comprises ≤ 10 per mL subvisible particles equal to or greater than 10 µm, and ≤ 2 per mL subvisible particles equal to or greater than 25 µm.

7. The drug product of any of the preceding claims wherein the drug product maintains the subvisible particle counts in elements (b)(iii) and (b)(iv) of claim 1 for one year when stored at 25 °C protected from light.

8. The drug product of any of the preceding claims wherein the solution comprises from 0.01 to 0.2 mg/ml noradrenaline bitartrate based on the weight of the free base.

9. The drug product of any of the preceding claims wherein the bag comprises two opposed walls sealed around their peripheries, wherein the walls comprise an interior ply of translucent or transparent polypropylene.

10. A hermetically sealed noradrenaline drug product in an intravenous ready to use translucent or transparent collapsible drip bag having low subvisible particle counts comprising:
a) a headspace consisting essentially of an inert gas, < 1.5% v/v oxygen immediately after the bag is hermetically sealed, and < 1.2% v/v after the oxygen in the headspace and solution have reached equilibrium; and
b) an aqueous liquid solution comprising:
i) noradrenaline or a pharmaceutically acceptable salt thereof at a concentration of from 0.001 to 0.2 mg/ml or from 0.001 to 0.035 mg/ml,
ii) a tonicifying effective amount of sodium chloride,
iii) hydrochloric acid in an amount effective to impart a pH of from 3.0 to 4.5,
iv) less than 200 ppbw oxygen immediately after the product has been hermetically sealed,
v) less than 500 ppbw oxygen after the oxygen in the solution and headspace have reached equilibrium,
vi) ≤ 25 per mL subvisible particles equal to or greater than 10 µm, and
vii) ≤ 3 per mL subvisible particles equal to or greater than 25 µm;
wherein:
i) the concentration of noradrenaline or pharmaceutically acceptable salt is based on the weight of the free base of noradrenaline;
ii) the subvisible particle count is determined by the light obscuration particle count test in USP <788> (May 1, 2013);
iii) the particle counts are observed immediately after the product is hermetically sealed and after the oxygen content in the headspace and solution has reached equilibrium; and
iv) the drug product is free of chelating agents and antioxidants.

11. The drug product of claim 10, wherein, immediately after the product is hermetically sealed,
a) the oxygen content in the headspace is less than 1.2% v/v; and
b) the oxygen content in the solution is less than 120 ppbw.

12. The drug product of claim 10 or 11 wherein, after the oxygen content in the solution and headspace has reached equilibrium,
a) the oxygen content in the headspace is less than 1.2% v/v; and
b) the oxygen content in the solution is less than 450 ppbw.

13. A method of making a hermetically sealed noradrenaline drug product in an intravenous ready to use translucent or transparent collapsible drip bag having low subvisible particle counts comprising:
a) dissolving noradrenaline or a pharmaceutically acceptable salt thereof in water to produce a noradrenaline liquid solution at a concentration of from 0.001 to 0.2 or from 0.001 to 0.035 mg/ml;
b) distributing the noradrenaline solution and an inert gas into the bag, such that the percentage of the bag's volume occupied by the headspace is from 0 to 25% or from 2 to 15%; and
c) hermetically sealing the bag;
wherein:
i) the oxygen concentration in the solution is less than 200 ppbw immediately after sealing the container and less than 500 ppbw after the oxygen content in the headspace and solution have reached equilibrium;
ii) the oxygen concentration in the headspace is less than 1.5 % immediately after sealing the container and less than 1.2% after the oxygen content in the headspace and solution have reached equilibrium;
iii) the drug product omits chelating agents, preservatives, and antioxidants; and
iv) when at equilibrium with the headspace, the oxygen content in the solution is less than 500 ppbw.

14. The method of claim 13 wherein,
a) immediately after the product is hermetically sealed, the oxygen content in the headspace is less than 1.2% v/v and the oxygen content in the solution is less than 120 ppbw; and
b) after the oxygen content in the solution and headspace has reached equilibrium, the oxygen content in the headspace is less than 1.2% v/v and the oxygen content in the solution is less than 450 ppbw.

15. The method of claim 13 or 14, undertaken in the presence of light without any extra precautions taken against the presence of light.

## Patentansprüche

1. Hermetisch versiegeltes Noradrenalin-Arzneistoffprodukt in einem intravenösen gebrauchsfertigen durchscheinenden oder transparenten faltbaren Tropfbeutel mit geringen Anzahlen subsichtbarer Partikel umfassend:
a) einen Kopfraum bestehend im Wesentlichen aus einem inerten Gas und < 2 % v/v Sauerstoff; und
b) eine flüssige Lösung umfassend:
i) Noradrenalin oder ein pharmazeutisch verträgliches Salz davon in einer Konzentration von 0,001 bis 0,2 mg/ml oder von 0,001 bis 0,035 mg/ml,
ii) weniger als 600 ppbw Sauerstoff,
iii) ≤ 25 pro mL subsichtbare Partikel, welche gleich oder größer als 10 µm sind, und
iv) ≤ 3 pro mL subsichtbare Partikel, welche gleich oder größer als 25 µm sind;
wobei:
i) die Konzentration an Noradrenalin oder pharmazeutisch verträglichem Salz auf das Gewicht der freien Base von Noradrenalin bezogen ist;
ii) die Anzahl subsichtbarer Partikel durch den Partikelanzahl-Lichttrübungstest in USP <788> (01. Mai 2013) bestimmt wird;
iii) der Sauerstoffgehalt in dem Kopfraum und der Lösung unmittelbar nachdem das Produkt hermetisch versiegelt wurde oder nachdem der Sauerstoffgehalt in dem Kopfraum und der Lösung Gleichgewicht erreicht hat gemessen wird;
iv) die Partikelanzahlen unmittelbar nachdem das Produkt hermetisch versiegelt wurde oder nachdem der Sauerstoffgehalt in dem Kopfraum und der Lösung Gleichgewicht erreicht hat beobachtet werden;
v) das Arzneistoffprodukt frei von Antioxidantien und chelatbildenden Mitteln ist.

2. Arzneistoffprodukt nach Anspruch 1, wobei das Volumen des Beutels 75 bis 550 ml oder 100 bis 350 ml oder 250 bis 325 ml beträgt, und der Prozentsatz des Volumens des Beutels, welches durch den Kopfraum eingenommen wird, 0 bis 25 % oder 2 bis 15 % beträgt.

3. Arzneistoffprodukt nach Anspruch 1 oder 2, wobei, unmittelbar nachdem das Produkt hermetisch versiegelt wurde,
a) der Sauerstoffgehalt in dem Kopfraum weniger als 1,5 % v/v oder 1,2 % v/v beträgt; und
b) der Sauerstoffgehalt in der Lösung weniger als 200 ppbw oder 120 ppbw beträgt.

4. Arzneistoffprodukt nach einem der vorhergehenden Ansprüche, wobei, unmittelbar nachdem das Produkt hermetisch versiegelt wurde, die Lösung ≤ 10 pro mL subsichtbare Partikel, welche gleich oder größer als 10 µm sind, und ≤ 2 pro mL subsichtbare Partikel, welche gleich oder größer als 25 µm sind, umfasst.

5. Arzneistoffprodukt nach einem der vorhergehenden Ansprüche, wobei, nachdem der Sauerstoffgehalt in dem Kopfraum und der Lösung Gleichgewicht erreicht hat,
a) der Sauerstoffgehalt in dem Kopfraum weniger als 1,5 % v/v beträgt; und
b) der Sauerstoffgehalt in der Lösung weniger als 500 ppbw beträgt.

6. Arzneistoffprodukt nach einem der vorhergehenden Ansprüche, wobei, unmittelbar nachdem der Sauerstoffgehalt in dem Kopfraum und der Lösung Gleichgewicht erreicht hat, die Lösung ≤ 10 pro mL subsichtbare Partikel, welche gleich oder größer als 10 µm sind, und ≤ 2 pro mL subsichtbare Partikel, welche gleich oder größer als 25 µm sind, umfasst.

7. Arzneistoffprodukt nach einem der vorhergehenden Ansprüche, wobei das Arzneistoffprodukt die Anzahlen subsichtbarer Partikel in Merkmalen (b)(iii) und (b)(iv) von Anspruch 1 ein Jahr lang aufrechterhält, wenn geschützt vor Licht bei 25°C gelagert wird.

8. Arzneistoffprodukt nach einem der vorhergehenden Ansprüche, wobei die Lösung 0,01 bis 0,2 mg/ml Noradrenalinbitartrat, bezogen auf das Gewicht der freien Base, umfasst.

9. Arzneistoffprodukt nach einem der vorhergehenden Ansprüche, wobei der Beutel zwei gegenüberliegende Wände, welche um ihre Peripherien herum versiegelt sind, umfasst, wobei die Wände eine innere Lage von durchscheinendem oder transparentem Polypropylen umfassen.

10. Hermetisch versiegeltes Noradrenalin-Arzneistoffprodukt in einem intravenösen gebrauchsfertigen durchscheinenden oder transparenten faltbaren Tropfbeutel mit geringen Anzahlen subsichtbarer Partikel umfassend:
a) einen Kopfraum bestehend im Wesentlichen aus einem inerten Gas, < 1,5 % v/v Sauerstoff unmittelbar nachdem der Beutel hermetisch versiegelt wurde, und < 1,2 % v/v nachdem der Sauerstoff in dem Kopfraum und der Lösung Gleichgewicht erreicht hat; und
b) eine wässrige flüssige Lösung umfassend:
i) Noradrenalin oder ein pharmazeutisch verträgliches Salz davon in einer Konzentration von 0,001 bis 0,2 mg/ml oder von 0,001 bis 0,035 mg/ml,
ii) eine Tonizität verleihende wirksame Menge an Natriumchlorid,
iii) Chlorwasserstoffsäure in einer Menge, welche zum Verleihen eines pH-Werts von 3,0 bis 4,5 wirksam ist,
iv) weniger als 200 ppbw Sauerstoff unmittelbar nachdem das Produkt hermetisch versiegelt wurde,
v) weniger als 500 ppbw Sauerstoff nachdem der Sauerstoff in der Lösung und dem Kopfraum Gleichgewicht erreicht hat,
vi) ≤ 25 pro mL subsichtbare Partikel, welche gleich oder größer als 10 µm sind, und
vii) ≤ 3 pro mL subsichtbare Partikel, welche gleich oder größer als 25 µm sind;
wobei:
i) die Konzentration an Noradrenalin oder pharmazeutisch verträglichem Salz auf das Gewicht der freien Base von Noradrenalin bezogen ist;
ii) die Anzahl subsichtbarer Partikel durch den Partikelanzahl-Lichttrübungstest in USP <788> (01. Mai 2013) bestimmt wird;
iii) die Partikelanzahlen unmittelbar nachdem das Produkt hermetisch versiegelt wurde und nachdem der Sauerstoffgehalt in dem Kopfraum und der Lösung Gleichgewicht erreicht hat beobachtet werden; und
iv) das Arzneistoffprodukt frei von chelatbildenden Mitteln und Antioxidantien ist.

11. Arzneistoffprodukt nach Anspruch 10, wobei, unmittelbar nachdem das Produkt hermetisch versiegelt wurde,
a) der Sauerstoffgehalt in dem Kopfraum weniger als 1,2 % v/v beträgt; und
b) der Sauerstoffgehalt in der Lösung weniger als 120 ppbw beträgt.

12. Arzneistoffprodukt nach Anspruch 10 oder 11, wobei, nachdem der Sauerstoffgehalt in der Lösung und dem Kopfraum Gleichgewicht erreicht hat,
a) der Sauerstoffgehalt in dem Kopfraum weniger als 1,2 % v/v beträgt; und
b) der Sauerstoffgehalt in der Lösung weniger als 450 ppbw beträgt.

13. Verfahren zur Herstellung eines hermetisch versiegelten Noradrenalin-Arzneistoffprodukts in einem intravenösen gebrauchsfertigen durchscheinenden oder transparenten faltbaren Tropfbeutel mit geringen Anzahlen subsichtbarer Partikel umfassend:
a) Lösen von Noradrenalin oder einem pharmazeutisch verträglichen Salz davon in Wasser, wobei eine flüssige Noradrenalin-Lösung in einer Konzentration von 0,001 bis 0,2 oder von 0,001 bis 0,035 mg/ml hergestellt wird;
b) Verteilen der Noradrenalin-Lösung und eines inerten Gases in dem Beutel, so dass der Prozentsatz des Volumens des Beutels, welches durch den Kopfraum eingenommen wird, 0 bis 25 % oder 2 bis 15 % beträgt; und
c) hermetisch Versiegeln des Beutels;
wobei:
i) die Sauerstoffkonzentration in der Lösung weniger als 200 ppbw unmittelbar nach dem Versiegeln des Behälters und weniger als 500 ppbw nachdem der Sauerstoffgehalt in dem Kopfraum und der Lösung Gleichgewicht erreicht hat beträgt;
ii) die Sauerstoffkonzentration in dem Kopfraum weniger als 1,5 % unmittelbar nach dem Versiegeln des Behälters und weniger als 1,2 % nachdem der Sauerstoffgehalt in dem Kopfraum und der Lösung Gleichgewicht erreicht hat beträgt;
iii) in dem Arzneistoffprodukt chelatbildende Mittel, Konservierungsstoffe, und Antioxidantien weggelassen werden; und
iv) wenn mit dem Kopfraum im Gleichgewicht, der Sauerstoffgehalt in der Lösung weniger als 500 ppbw beträgt.

14. Verfahren nach Anspruch 13, wobei,
a) unmittelbar nachdem das Produkt hermetisch versiegelt wurde, der Sauerstoffgehalt in dem Kopfraum weniger als 1,2 % v/v beträgt und der Sauerstoffgehalt in der Lösung weniger als 120 ppbw beträgt; und
b) nachdem der Sauerstoffgehalt in der Lösung und dem Kopfraum Gleichgewicht erreicht hat, der Sauerstoffgehalt in dem Kopfraum weniger als 1,2 % v/v beträgt und der Sauerstoffgehalt in der Lösung weniger als 450 ppbw beträgt.

15. Verfahren nach Anspruch 13 oder 14, durchgeführt in der Gegenwart von Licht ohne jedwede zusätzlichen Vorkehrungen, welche gegen die Gegenwart von Licht ergriffen werden.

## Revendications

1. Produit pharmaceutique à base de noradrénaline hermétiquement scellé dans une poche de goutte-à-goutte intraveineux pliable, translucide ou transparente, prête à l'emploi, à faible nombre de particules subvisibles comprenant :
a) un espace de tête constitué essentiellement d'un gaz inerte et de < 2% v/v d'oxygène ; et
b) une solution liquide comprenant :
i) de la noradrénaline ou un sel pharmaceutiquement acceptable de celle-ci à une concentration de 0,001 à 0,2 mg/ml ou de 0,001 à 0,035 mg/ml,
ii) moins de 600 ppb en poids d'oxygène,
iii) ≤ 25 par mL de particules subvisibles supérieures ou égales à 10 µm, et
iv) ≤ 3 par mL de particules subvisibles supérieures ou égales à 25 µm ;
où :
i) la concentration de noradrénaline ou de sel pharmaceutiquement acceptable est basée sur le poids de la base libre de noradrénaline ;
ii) le nombre de particules subvisibles est déterminé par le test de comptage de particules par obscurcissement de la lumière dans USP <788> (1er mai 2013) ;
iii) la teneur en oxygène dans l'espace de tête et la solution est mesurée immédiatement après que le produit est hermétiquement scellé ou après que la teneur en oxygène dans l'espace de tête et la solution a atteint l'équilibre ;
iv) le nombre de particules est observé immédiatement après que le produit est hermétiquement scellé ou après que la teneur en oxygène dans l'espace de tête et la solution a atteint l'équilibre ;
v) le produit pharmaceutique est exempt d'antioxydants et d'agents chélatants.

2. Produit pharmaceutique de la revendication 1, dans lequel le volume de la poche est de 75 à 550 ml ou de 100 à 350 ml ou de 250 à 325 ml, et le pourcentage du volume de la poche occupé par l'espace de tête est de 0 à 25% ou de 2 à 15%.

3. Produit pharmaceutique de la revendication 1 ou 2, dans lequel, immédiatement après que le produit est hermétiquement scellé,
a) la teneur en oxygène dans l'espace de tête est inférieure à 1,5% v/v ou 1,2% v/v ; et
b) la teneur en oxygène dans la solution est inférieure à 200 ppb en poids ou 120 ppb en poids.

4. Produit pharmaceutique de l'une des revendications précédentes, dans lequel, immédiatement après que le produit est hermétiquement scellé, la solution comprend ≤ 10 par mL de particules subvisibles supérieures ou égales à 10 µm, et ≤ 2 par mL de particules subvisibles supérieures ou égales à 25 µm.

5. Produit pharmaceutique de l'une des revendications précédentes, dans lequel, après que la teneur en oxygène dans l'espace de tête et la solution a atteint l'équilibre,
a) la teneur en oxygène dans l'espace de tête est inférieure à 1,5% v/v ; et
b) la teneur en oxygène dans la solution est inférieure à 500 ppb en poids.

6. Produit pharmaceutique de l'une des revendications précédentes, dans lequel, immédiatement après que la teneur en oxygène dans l'espace de tête et la solution a atteint l'équilibre, la solution comprend ≤ 10 par mL de particules subvisibles supérieures ou égales à 10 µm et ≤ 2 par mL de particules subvisibles supérieures ou égales à 25 µm.

7. Produit pharmaceutique de l'une des revendications précédentes, dans lequel le produit pharmaceutique maintient le nombre de particules subvisibles dans les éléments (b)(iii) et (b)(iv) de la revendication 1 pendant un an lorsqu'il est stocké à 25°C à l'abri de la lumière.

8. Produit pharmaceutique de l'une des revendications précédentes, dans lequel la solution comprend de 0,01 à 0,2 mg/ml de bitartrate de noradrénaline par rapport au poids de la base libre.

9. Produit pharmaceutique de l'une des revendications précédentes, dans lequel la poche comprend deux parois opposées scellées autour de leurs périphéries, dans lequel les parois comprennent un pli intérieur de polypropylène translucide ou transparent.

10. Produit pharmaceutique de noradrénaline hermétiquement scellé dans une poche de goutte-à-goutte intraveineux pliable, translucide ou transparente, prête à l'emploi ayant un faible nombre de particules subvisibles comprenant :
a) un espace de tête constitué essentiellement d'un gaz inerte, < 1,5% v/v d'oxygène immédiatement après que la poche est hermétiquement scellée, et < 1,2% v/v après que l'oxygène dans l'espace de tête et la solution a atteint l'équilibre ; et
b) une solution liquide aqueuse comprenant :
i) de la noradrénaline ou un sel pharmaceutiquement acceptable de celle-ci à une concentration de 0,001 à 0,2 mg/ml ou de 0,001 à 0,035 mg/ml,
ii) une quantité efficace tonifiante de chlorure de sodium,
iii) de l'acide chlorhydrique en une quantité efficace pour conférer un pH de 3,0 à 4,5,
iv) moins de 200 ppb en poids d'oxygène immédiatement après que le produit a été hermétiquement scellé,
v) moins de 500 ppb en poids d'oxygène après que l'oxygène dans la solution et l'espace de tête a atteint l'équilibre,
vi) ≤ 25 par mL de particules subvisibles supérieures ou égales à 10 µm, et
vii) ≤ 3 par mL de particules subvisibles supérieures ou égales à 25 µm ;
où :
i) la concentration de noradrénaline ou de sel pharmaceutiquement acceptable est basée sur le poids de la base libre de noradrénaline ;
ii) le nombre de particules subvisibles est déterminé par le test de comptage de particules par obscurcissement de la lumière dans USP <788> (1er mai 2013) ;
iii) le nombre de particules est observé immédiatement après que le produit est hermétiquement scellé et après que la teneur en oxygène dans l'espace de tête et la solution a atteint l'équilibre ; et
iv) le produit pharmaceutique est exempt d'agents chélatants et d'antioxydants.

11. Produit pharmaceutique de la revendication 10, dans lequel, immédiatement après que le produit est hermétiquement scellé,
a) la teneur en oxygène dans l'espace de tête est inférieure à 1,2% v/v ; et
b) la teneur en oxygène dans la solution est inférieure à 120 ppb en poids.

12. Produit pharmaceutique de la revendication 10 ou 11, dans lequel, après que la teneur en oxygène dans la solution et l'espace de tête a atteint l'équilibre,
a) la teneur en oxygène dans l'espace de tête est inférieure à 1,2% v/v ; et
b) la teneur en oxygène dans la solution est inférieure à 450 ppb en poids.

13. Procédé de fabrication d'un produit pharmaceutique de noradrénaline hermétiquement scellé dans une poche de goutte-à-goutte intraveineux, pliable translucide ou transparente, prête à l'emploi ayant un faible nombre de particules subvisibles, comprenant :
a) la dissolution de la noradrénaline ou d'un sel pharmaceutiquement acceptable de celle-ci dans l'eau pour produire une solution liquide de noradrénaline à une concentration de 0,001 à 0,2 ou de 0,001 à 0,035 mg/ml ;
b) la distribution de la solution de noradrénaline et d'un gaz inerte dans la poche, de sorte que le pourcentage du volume de la poche occupé par l'espace de tête soit de 0 à 25% ou de 2 à 15% ; et
c) le scellement hermétique de la poche ;
où :
i) la concentration en oxygène dans la solution est inférieure à 200 ppb en poids immédiatement après le scellement hermétique du récipient et inférieure à 500 ppb en poids après que la teneur en oxygène dans l'espace de tête et la solution a atteint l'équilibre ;
ii) la concentration en oxygène dans l'espace de tête est inférieure à 1,5% immédiatement après le scellement du récipient et inférieure à 1,2% après que la teneur en oxygène dans l'espace de tête et la solution a atteint l'équilibre ;
iii) le produit pharmaceutique ne contient pas d'agents chélatants, de conservateurs et d'antioxydants ; et
iv) lorsqu'elle est à l'équilibre avec l'espace de tête, la teneur en oxygène dans la solution est inférieure à 500 ppb en poids.

14. Procédé de la revendication 13 dans lequel,
a) immédiatement après que le produit est hermétiquement scellé, la teneur en oxygène dans l'espace de tête est inférieure à 1,2% v/v et la teneur en oxygène dans la solution est inférieure à 120 ppb en poids ; et
b) après que la teneur en oxygène dans la solution et l'espace de tête a atteint l'équilibre, la teneur en oxygène dans l'espace de tête est inférieure à 1,2% v/v et la teneur en oxygène dans la solution est inférieure à 450 ppb en poids.

15. Procédé de la revendication 13 ou 14, mis en oeuvre en présence de lumière sans aucune précaution supplémentaire prise contre la présence de lumière.
